# EUROPEAN PATENT SPECIFICATION

(11) **EP 4 340 695 B1**
(45) Date of publication and mention of the grant of the patent: **19.08.2026**
(21) Application number: 22729674.6
(22) Date of filing: 19.05.2022
(51) Int. Cl.: A61B 1/005, A61B 1/00, A61B 1/31

(54) **AN ENDOSCOPE WITH AN ERGONOMIC HANDLE**
ENDOSKOP MIT ERGONOMISCHEM GRIFF
ENDOSCOPE À POIGNÉE ERGONOMIQUE

(30) Priority: 20.05.2021 DK PA202170256
(43) Date of publication of application: 27.03.2024
(73) Proprietor: Ambu A/S, 2750 Ballerup (DK)
(72) Inventor: QUENSEL, Ulf, 2750 Ballerup (DK); BESANA, Andrea, 2750 Ballerup (DK); EDWARDSSON, Henrik, 2750 Ballerup (DK); TORNAGHI, Barbara, 2750 Ballerup (DK); NERSING, Lovisa, 2750 Ballerup (DK); FORNANDER, Mårten, 2750 Ballerup (DK)
(74) Representative: AWA Denmark A/S
(86) International application number: PCT/EP2022/063627
(87) International publication number: WO 2022/243465

(56) References cited:
- WO-A1-2018/162561
- WO-A1-2020/070775
- WO-A1-2020/150709

## Description

The present disclosure relates to an endoscope with an ergonomic handle, preferably but not exclusively an endoscope for colonoscopy or gastroscopy, such as a duodenoscope for endoscopic retrograde cholangiopan-creatography (ERCP).

When performing colonoscopy on a patient, the patient will normally be lying down in a supine or prone position. The medical professional will be standing holding the endoscope in the left hand during insertion, and upon initial insertion be focussed away from the patient i.e. looking at a display device showing the images recorded by the endoscope, while manoeuvring the tip of the endoscope, using the right hand to turn one or often two knobs in order to bend a highly flexible bending section at the distal end of the insertion cord of the endoscope in two or four directions, while advancing the insertion cord through a series of turns and bends to the desired location inside the patient.

This may result in movements that may be somewhat awkward for the medical professional. There is therefore a need for the endoscope to be ergonomically supportive for such manoeuvring. This *inter alia* necessitates that the transition from the handle to the insertion cord is quite rigid so that the proximal part of the insertion cord remains a straight extension of the handle, i.e. does not bend or kink close to the location where the insertion cord exits from the handle.

In this respect, WO2020/074913 discloses an endoscope for sigmoid coloscopy which is adapted for single handed use. More specifically, the endoscope comprises a handle with a proximal gripping section, a distal connector for the insertion cord and an intermediate manipulation section. The proximal gripping section is bent downward at an angle somewhat like a pistol grip or a rifle stock when the insertion cord is held in an initial horizontal position.

As to pistol grip versus rifle stock, WO2018/162423 shows a somewhat similar endoscope with a handle having a proximal gripping section, an intermediate manipulation section and an insertion cord extending from the distal end of the handle. That is to say, a similar approach only with a much larger angle approaching almost a right angle between the insertion cord and the gripping section.

WO2018/162561 discloses a disposable bronchoscope also adapted for single handed use. The handle of this bronchoscope is gripped in an entirely different manner from that of WO2020/074913 where he natural initial position is with the insertion cord pointing vertically downward from the handle. Accordingly, the handle of WO2018/162561 has a proximal manipulation section at the top, an intermediate gripping section and a distal handle closing part at the bottom from which the insertion cord extends.

If for the sake of comparison the operator of an endoscope according to WO2020/074913, i.e. the medical professional, were to hold the insertion cord pointing vertically downward the proximal end of the handle would have an inclination towards the operator to allow the entire arm with the hand and the thumb to also be pointing downward. On the other hand, in the case of an endoscope according to WO2018/162561 the lower arm, the hand and the thumb would be horizontal.

These two handles thus represent two entirely different approaches to gripping which would seem incompatible with each other.

WO2020/150709 shows a catheter with a curved handle. The requirements for the insertion of the catheter differ in many ways from those of an endoscope. In particular endoscopes for colonoscopy or gastroscopy, such as duodenoscopes for endoscopic retrograde cholangiopancreatog-raphy (ERCP), as recited in the opening paragraph, comprise quite rigid insertion cords as compared to the catheter disclosed in WO2020/150709. This is *inter alia* in order to allow good control of the angle through which the tip is turned during insertion manoeuvring. Furthermore, the transition from the handle to the insertion cord need to be more rigid than the bendable transition illustrated in WO2020/150709.

Moreover, the bending section in WO2020/150709 narrows down towards the operating section provided as a head at the proximal end of the handle. This reduces the efficiency of the grip when it comes to torque transfer, much in the same manner as a large screwdriver handle provides more torque turning a screw than a screwdriver with a smaller handle does.

Based on this prior art is an object to provide an endoscope with an ergonomically improved handle, in particular but not exclusively facilitating and supporting motions of an operator during colonoscopy procedures.

According to a first aspect of the disclosure this object is achieved by an endoscope with an ergonomic handle and an insertion cord with a longitudinal centre axis in the straight as-made state, where the ergonomic handle comprises a proximal manipulation section, an intermediate gripping section and a distal transition section from which the insertion cord extends at the bottom of the handle in the normal holding position for an endoscopic procedure, where the gripping section comprises an outer circumferential surface adapted to be gripped by a middle finger, a ring finger and a little finger of one hand of an operator, in such a manner that the little finger is closer to the distal transition section, and the middle finger is closer to the proximal manipulation section, where said outer circumferential gripping surface comprises a front surface area and a back surface area, wherein the front surface is inclined forwardly as seen in the upward direction from the transition section towards the manipulation section at a front forward angle with respect to a left to right vertical plane in which the centre line of the insertion cord lies, and wherein the back surface is inclined forwardly as seen in the upward direction from the transition section towards the manipulation section at a back forward angle with respect to said vertical plane, characterized in that the front forward angle is larger than the back forward angle.

Providing the handle with an angled gripping section in this manner allows a better initial posture of the operator before and during the insertion of the endoscope. It also provides the operator with a better grip in the sense of allowing better torque transfer to the insertion cord via the handle. As a bonus effect more space is provided within the handle for *inter alia* an operating mechanism for a four-way bending section as usually employed in endoscopes of the kind mentioned in the opening paragraph. Further things that may also find place could be additional operating mechanisms, such as for an Albarran lever, as well as valves and suction tubes within the handle.

According to a second aspect of the disclosure the problem is solved by a system comprising a display device and an endoscope according to the first aspect of the disclosure connectable to the display device.

The handle with the angled gripping section facilitates this, as the angle allows for different better postures of the operator who, after initial insertion of the endoscope will be turned away from the patent focussing instead on the display device.

According to an embodiment of the first aspect of the disclosure the back forward angle at which the back surface area extends forwardly comprises an angle between 10° and 20°, preferably 14°, with respect to a vertical left to right plane in which the centre axis of insertion cord lies. This has been found to provide a good grip and improving the angle of the operator's hand during initial insertion and throughout the procedure.

According to a preferred embodiment, the front surface area comprises a saddle shape. In this respect saddle shape is generally being understood as a geometrical surface with a concave shape in one direction and a convex shape in the crosswise direction. With a saddle shape where the forward curvature is concave in the longitudinal direction and the circumference of the handle provides the convex shape, this provides good grip for the three fingers with which the gripping section is intended to be normally gripped with.

According to a further embodiment, the back surface area comprises an essentially flat surface. This provides good rest for the relevant part of the palm of the operator's hand while providing good access to image capture buttons or the like arranged at or close to the top of the manipulation section.

According to another preferred embodiment, the manipulation section comprises at least one part protruding forwardly from the gripping section so as to allow the handle to rest on top of the middle finger of the operator. This reduces the necessity for a firm grip, in turn allowing the operator's grip and hand to be more relaxed, thus also improving ergonomics.

According to a further preferred embodiment, the length of the back surface area is approximately 100 mm or more. This allows for variation in the way different operators may grip the handle as well as for differences in their hand size.

The invention will now be described in greater detail based on nonlimiting exemplary embodiments and with reference to the drawings, on which:
Fig. 1 shows a system with an endoscope according to the disclosure connected to a display device via an umbilical,
Fig. 2 shows the handle of the endoscope of Fig. 1 in isometric view
Fig. 3 shows a side view of the endoscope of Fig. 1 for comparison with
Fig. 4 showing the ergonomically improved grip on the handle.

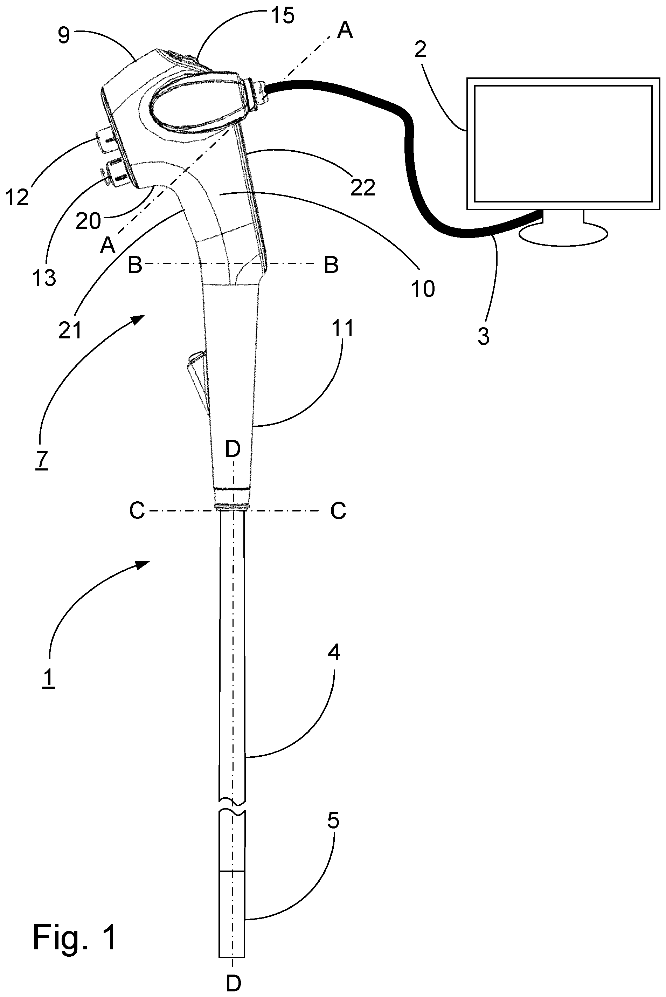

Turning now to Fig. 1 a system comprising an endoscope 1 and a display device 2. In the illustrated system the endoscope 1 is connected to the display via a cable connection 3, but for display purposes wireless could also be used. In a typical setup the cable connection 3 would be a part of an umbilical, also serving as supply for water and/or air, electrical power, suction, etc.

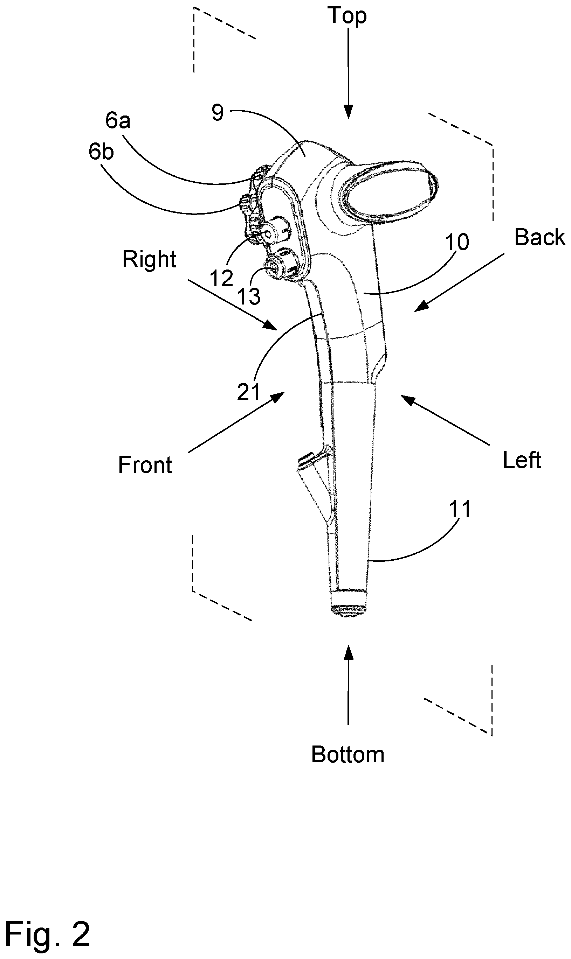

The endoscope 1 comprises a proximal handle 7 and an insertion cord 4 with a controllable bending section 5 at the distal end. In the illustrated embodiment the bending section may be bent in four directions, viz. forward, backward, left and right under control of two control knobs 6a, 6b arranged on the handle 7 of the endoscope, visible in Fig. 2.

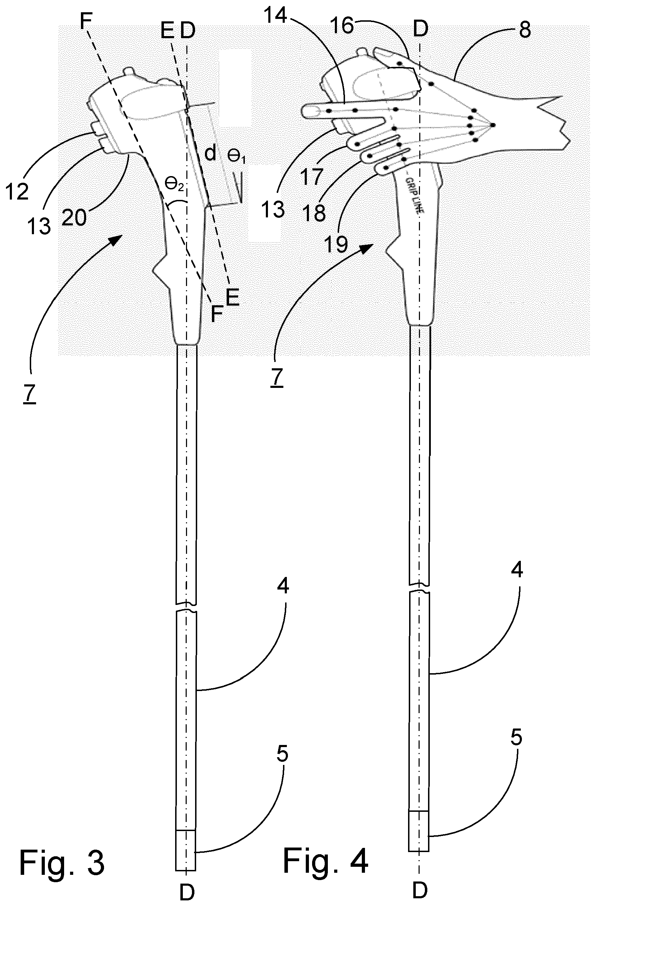

Orientations relating to the handle 7 and to the endoscope 1 in general are in this description and in the claims defined and to be understood as shown in Fig. 2. That is to say, referring to the endoscope 1 in a position and orientation as held in the hand 8 of the operator in front of the operator with the insertion cord pointing vertically down as shown in Fig. 4.

As can be seen in Fig. 1 the handle comprises three sections, viz. a proximal manipulation section 9 above the dashed line A-A, an intermediate gripping section 10 between the dashed line B-B and the dashed line A-A, and a distal transition section 11 between the dashed line B-B and the dashed line C-C where the insertion cord 4 extends from the distal end of the handle 7.

As mentioned, the manipulation section 9 comprises control knobs 6a, 6b for controlling the bending section. The handle 7 according to the illustrated embodiment is intended to be gripped by the left hand 8 of the operator as shown in Fig. 4. Consequently, the knobs 6a, 6b are arranged at the righthand side of the manipulation section so as to be easily accessible by the right hand of the operator. The manipulation section 9 may further comprise control buttons 12, 13 arranged on the front of the manipulation section 9 for irrigation, suction or the like. The control buttons 12, 13 being so positioned that they can be easily reached by the index finger 14 of the hand 8 with which the operator is gripping the gripping section 10. The manipulation section may also comprise one or more actuator buttons 15 for other functions such as image capture arranged at the back of the manipulation section 9 so as to be easily depressed by the thumb 16 of the hand 8 with which the operator is gripping the gripping section 10.

The surface of the gripping section 10 below the manipulation section 9 is adapted to be gripped by the palm and the remaining three fingers of the hand 8, as can be seen in Fig. 4. The circumference of the gripping section 10 decreases from the manipulation section 9 towards the transition section 11, so as to take into account the fact that the middle finger 17 is normally longer than the ring finger 18, in turn being longer than the little finger 19. The handle 7 is thus designed specifically for a grip where the little finger 19 is gripping the gripping section more distally than the middle finger 17 and the ring finger 18. In this respect it should also be noted that the manipulation section 9 protrudes forwardly from the gripping section 10 and presents a lower surface 20 adapted to rest on the middle finger 17.

The front surface area 21 of the gripping section 10 has a curvature as seen in projection from the side and as can also be seen inclined forwardly in the upward direction from the transition section 11 towards the manipulation section 9 as indicated by the dashed line E-E in Fig. 3. The gripping section 11 is preferably somewhat ovoid in cross-section with the pointed end in the forward direction, and accordingly the front surface area 21 may accordingly have a saddle shape if, as preferred, the forward curvature is concave as illustrated.

The back surface area 22 has a curvature and is adapted to be engaged by parts of the palm of the gripping hand 8. The back surface area 22 can be rounded in a way similar to the front surface area 21, but will preferably also comprise a generally straight section as seen from the side. The back surface area 22 is also inclined forwardly as seen in the upward direction from the transition section 11 towards the manipulation section 9. That is, as illustrated by the dashed line E-E in Fig. 3, inclined at a second forward angle Θ₁ with respect to a left to right vertical plane in which the centre line D-D of the insertion cord 4 lies, as illustrated by the dashed corners in Fig. 2. The back surface has some curvature, be it a convex shape in the forward direction or a rounded, more ovoid cross-sectional shape. To accommodate for various hand sizes, the length d of the back surface area 22 is preferably approximately 100 mm or more.

The back forward inclination Θ₁ of back surface area 22 is preferably between 10° and 20°, and more preferred 14°, these values applying to the tangent to the forward curvature intersecting the left to right vertical plane. The forward inclination angle Θ₂ of the straight part of the front gripping surface 21, also referred to as front forward angle Θ₂, as illustrated by the dashed line F-F again with respect to a left to right vertical plane in which the centre line D-D of the insertion cord 4 lies is higher than the back forward angle Θ₁ so as provide the aforementioned decrease in circumference towards the distal end. As illustrated this decrease in circumference is preferably continuous and monotonous along the majority, if not the entire, length of the gripping section 10 from the operating section 9 to the transition section 11. In any case the continuous and monotonous decrease would at least be found between the straight sections of the front gripping surface 21 and the back surface 22 area, having the respective front forward and front backward angles Θ₂ and Θ₁. The forward inclination Θ₂ of the front gripping surface 21 applies to the tangent to the forward curvature intersecting the left to right vertical plane. The same applies to the back surface. The tangent defining the forward front angle Θ₂ is larger than the tangent defining the back forward angle Θ₁ at corresponding locations on the gripping section. That is to say opposite, as defined where the cross-section of the handle gripping section taken between the tangent points is at a minimum.

Since both the front surface area 21 and the back surface area 22 are inclined the handle 7 essentially has a curvature or a bend that allows the gripping of the handle 7 at an overall angle to the centre line D-D of the insertion cord 4, thereby improving the ergonomics for the operator. That is to say, advantageously with a grip line, i.e. an imaginary line intersecting the knuckles of the middle finger, a ring finger and a little finger of the gripping hand 8 that advantageously deviates centre line D-D of the insertion tube 4.

## Claims

1. An endoscope (1) with an ergonomic handle (7) and an insertion cord (4) with a longitudinal centre axis (D) in the straight as-made state,
where the ergonomic handle (7) comprises a proximal manipulation section (9), an intermediate gripping section (10) and a distal transition section (11) from which the insertion cord (4) extends at the bottom of the handle (7) in the normal holding position,
where the gripping section (10) comprises an outer circumferential surface adapted to be gripped by a middle finger, a ring finger and a little finger of one hand of an operator, in such a manner that the little finger is closer to the distal transition section (11), and the middle finger is closer to the proximal manipulation section (9),
where said outer circumferential gripping surface comprises a front surface area (21) and a back surface area (22), wherein the back surface area (22) is adapted to be engaged by the palm of the gripping hand (8), and the front surface area (21) is adapted to be engaged by the middle finger, the ring finger and the little finger,
wherein the front surface area (21) has a forward curvature and is inclined forwardly as seen in the upward direction from the transition section (11) towards the manipulation section (9) at a front forward angle (Θ₂) with respect to a left to right vertical plane in which the centre line (D) of the insertion cord (4) lies, and
wherein the back surface area (22) has a forward curvature and is inclined forwardly as seen in the upward direction from the transition section (11) towards the manipulation section (9) at a back forward angle (Θ₁) with respect to said vertical plane, **charac- terized** in that the front forward angle (Θ₂) is larger than the back forward angle (Θ₁), the forward front angle (Θ₂) and the back forward angle (Θ₁) being defined as the tangents at opposite corresponding tangent point locations on the gripping section (10) where the cross-section of the handle gripping section (10) taken between the tangent points is at a minimum.

2. An endoscope (1) according to claim 1, wherein the back forward angle (Θ₁) at which the back surface area (22) extends forwardly comprises an angle between 10° and 20°, preferably 14°, with respect to a vertical left to right plane in which the centre axis (D) of the insertion cord (4) lies.

3. An endoscope (1) according to any one of the preceding claims, wherein said front surface area (21) comprises a saddle shape.

4. An endoscope (1) according to any one of the preceding claims wherein the manipulation section (9) comprises at least one part protruding forwardly from the gripping section (10) so as to allow the handle (7) to rest on top of the middle finger of the operator.

5. An endoscope (1) according to any one of the preceding claims wherein the length of the back surface area (22) is approximately 100 mm or more.

6. System comprising a display device (2) and an endoscope (1) according to any one of the preceding claims connectable to the display device (2).

## Patentansprüche

1. Endoskop (1) mit einem ergonomischen Griff (7) und einem Einführkabel (4) mit einer Längsmittelachse (D) im geraden Herstellzustand,
wobei der ergonomische Griff (7) einen proximalen Manipulationsabschnitt (9), einen Zwischengreifabschnitt (10) und einen distalen Übergangsabschnitt (11) umfasst, von dem sich das Einführkabel (4) an der Unterseite des Griffs (7) in der normalen Halteposition erstreckt,
wobei der Greifabschnitt (10) eine Außenumfangsfläche umfasst, die dazu ausgeführt ist, so von einem Mittelfinger, einem Ringfinger und einem kleinen Finger einer Hand einer Bedienperson ergriffen zu werden, dass sich der kleine Finger näher an dem distalen Übergangsabschnitt (11) befindet und sich der Mittelfinger näher an dem proximalen Manipulationsabschnitt (9) befindet,
wobei die Außenumfangsgreiffläche einen vorderen Flächenbereich (21) und einen hinteren Flächenbereich (22) umfasst, wobei der hintere Flächenbereich (22) dazu ausgeführt ist, von der Handfläche der Greifhand (8) in Eingriff genommen zu werden, und der vordere Flächenbereich (21) dazu ausgeführt ist, von dem Mittelfinger, dem Ringfinger und dem kleinen Finger in Eingriff genommen zu werden,
wobei der vordere Flächenbereich (21) eine Vorwärtskrümmung aufweist und in der Aufwärtsrichtung gesehen von dem Übergangsabschnitt (11) zu dem Manipulationsabschnitt (9) in einem vorderen Vorwärtswinkel (Θ₂) in Bezug auf eine von links nach rechts gehende vertikale Ebene, in der die Mittellinie (D) des Einführkabels (4) liegt, nach vorne geneigt ist, und
wobei der hintere Flächenbereich (22) eine Vorwärtskrümmung aufweist und in der Aufwärtsrichtung gesehen von dem Übergangsabschnitt (11) zu dem Manipulationsabschnitt (9) in einem hinteren Vorwärtswinkel (Θ₁) in Bezug auf die vertikale Ebene nach vorne geneigt ist, **dadurch gekennzeichnet, dass** der vordere Vorwärtswinkel (Θ₂) größer als der hintere Vorwärtswinkel (Θ₁) ist, wobei der vordere Vorwärtswinkel (Θ₂) und der hintere Vorwärtswinkel (Θ₁) als die Tangenten an gegenüberliegenden entsprechenden Tangentialpunktstellen an dem Greifabschnitt (10) definiert sind, wobei der Querschnitt des Greifabschnitts (10) zwischen den Tangentialpunkten minimal ist.

2. Endoskop (1) nach Anspruch 1, wobei der hintere Vorwärtswinkel (Θ₁), unter dem sich der hintere Flächenbereich (22) nach vorne erstreckt, einen Winkel zwischen 10° und 20°, vorzugsweise 14°, in Bezug auf eine vertikale von links nach rechts gehende Ebene umfasst, in der die Mittelachse (D) des Einführkabels (4) liegt.

3. Endoskop (1) nach einem der vorhergehenden Ansprüche, wobei der vordere Flächenbereich (21) eine Sattelform umfasst.

4. Endoskop (1) nach einem der vorhergehenden Ansprüche, wobei der Manipulationsabschnitt (9) mindestens einen Teil umfasst, der von dem Greifabschnitt (10) nach vorne vorsteht, damit der Griff (7) auf dem Mittelfinger der Bedienperson aufliegen kann.

5. Endoskop (1) nach einem der vorhergehenden Ansprüche, wobei die Länge des hinteren Flächenbereichs (22) ungefähr 100 mm oder mehr beträgt.

6. System, umfassend eine Anzeigevorrichtung (2) und ein Endoskop (1) nach einem der vorhergehenden Ansprüche, das mit der Anzeigevorrichtung (2) verbindbar ist.

## Revendications

1. Endoscope (1) muni d'une poignée ergonomique (7) et d'un cordon d'insertion (4) avec un axe central longitudinal (D) à l'état droit brut de fabrication,
la poignée ergonomique (7) comprenant une section de manipulation proximale (9), une section de préhension intermédiaire (10) et une section de transition distale (11) à partir de laquelle le cordon d'insertion (4) s'étend au fond de la poignée (7) dans la position de maintien normale,
la section de préhension (10) comprenant une surface circonférentielle extérieure adaptée pour être saisie par un majeur, un annulaire et un auriculaire d'une main d'un opérateur, de manière que l'auriculaire soit plus proche de la section de transition distale (11), et que le majeur soit plus proche de la section de manipulation proximale (9),
ladite surface circonférentielle extérieure de préhension comprenant une zone de surface avant (21) et une zone de surface arrière (22), la zone de surface arrière (22) étant adaptée pour être engagée par la paume de la main de préhension (8), et la zone de surface avant (21) étant adaptée pour être engagée par le majeur, l'annulaire et l'auriculaire,
la zone de surface avant (21) présentant une courbure vers l'avant et étant inclinée vers l'avant, vue vers le haut, de la section de transition (11) vers la section de manipulation (9), à un angle vers l'avant avant (Θ₂) par rapport à un plan vertical gauche-droite dans lequel se trouve l'axe central (D) du cordon d'insertion (4), et
la zone de surface arrière (22) présentant une courbure vers l'avant et étant inclinée vers l'avant, vue dans la direction vers le haut, depuis la section de transition (11) vers la section de manipulation (9) selon un angle vers l'avant arrière (Θ₁) par rapport audit plan vertical, **caractérisé en ce que** l'angle vers l'avant avant (Θ₂) est supérieur à l'angle vers l'avant arrière (Θ₁), l' angle vers l'avant avant (Θ₂) et l'angle vers l'avant arrière (Θ₁) étant définis comme les tangentes à des points tangents correspondants opposés sur la section de préhension (10) où la section transversale de la section de préhension de poignée (10) prise entre les points tangents est minimale.

2. Endoscope (1) selon la revendication 1, l'angle avant arrière (Θ₁) auquel la zone de surface arrière (22) s'étend vers l'avant comprenant un angle compris entre 10° et 20°, de préférence 14°, par rapport à un plan vertical gauche à droite dans lequel se trouve l'axe central (D) du cordon d'insertion (4).

3. Endoscope (1) selon l'une quelconque des revendications précédentes, ladite zone de surface avant (21) comprenant une forme de selle.

4. Endoscope (1) selon l'une quelconque des revendications précédentes, la section de manipulation (9) comprenant au moins une partie faisant saillie vers l'avant à partir de la section de préhension (10) de manière à permettre à la poignée (7) de reposer sur le dessus du majeur de l'opérateur.

5. Endoscope (1) selon l'une quelconque des revendications précédentes, la longueur de la zone de surface arrière (22) étant d'environ 100 mm ou plus.

6. Système comprenant un dispositif d'affichage (2) et un endoscope (1) selon l'une quelconque des revendications précédentes, pouvant être connecté au dispositif d'affichage (2).
